(19) **Europäisches Patentamt / European Patent Office / Office européen des brevets**

(11) **EP 3 531 972 B1**

(12) **FASCICULE DE BREVET EUROPEEN**

(45) Date de publication et mention de la délivrance du brevet:
**07.12.2022 Bulletin 2022/49**

(21) Numéro de dépôt: **17791173.2**

(22) Date de dépôt: **18.10.2017**

(51) Classification Internationale des Brevets (IPC):
***A61F 2/12*** *(2006.01)*

(52) Classification Coopérative des Brevets (CPC):
**A61F 2/12**

(86) Numéro de dépôt international:
**PCT/IB2017/056464**

(87) Numéro de publication internationale:
**WO 2018/078489 (03.05.2018 Gazette 2018/18)**

(54) **DISPOSITIF APTE A ETRE IMPLANTE DANS LE CORPS D'UN SUJET POUR FORMER UN IMPLANT**

IM KÖRPER EINER PERSON ZU IMPLANTIERBARE VORRICHTUNG ZUR FORMUNG EINES IMPLANTATS

DEVICE TO BE IMPLANTED IN A SUBJECT'S BODY TO FORM AN IMPLANT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**MA MD**

(30) Priorité: **28.10.2016 FR 1670642**

(43) Date de publication de la demande:
**04.09.2019 Bulletin 2019/36**

(73) Titulaires:
• **Centre Hospitalier Regional Universitaire de Lille**
**59037 Lille (FR)**
• **Université de Lille**
**59800 Lille (FR)**
• **Payen, Julien**
**59134 Fournes en Weppes (FR)**

(72) Inventeurs:
• **DANZE, Pierre-Marie**
**59037 Lille (FR)**
• **MARCHETTI, Philippe**
**59037 Lille (FR)**
• **GUERRESCHI, Pierre**
**59037 Lille (FR)**
• **PAYEN, Julien**
**59134 Fournes en Weppes (FR)**

(74) Mandataire: **Ipside**
**29, rue de Lisbonne**
**75008 Paris (FR)**

(56) Documents cités:
**WO-A1-01/66039          WO-A2-2012/122215**
**US-A- 5 545 217          US-A1- 2013 253 646**
**US-A1- 2014 135 925**

EP 3 531 972 B1

**Description**

**[Domaine technique]**

La présente invention concerne un dispositif apte à être implanté dans le corps d'un sujet pour former un implant apte à remplacer et/ou augmenter un volume de tissu mou, une masse tissulaire et un procédé de fabrication dudit implant.

**[Art Antérieur]**

Les implants mammaires comprenant une poche remplie d'un gel de silicone sont couramment utilisées. Néanmoins, il existe des risques de rupture de la poche qui ne sont pas négligeables. On a donc cherché des alternatives à ces implants mammaires.

Ainsi, le document WO 01/66039 A1 décrit une coque poreuse qui comprend plusieurs couches de textile dans le volume interne qu'elle définit. La coque est conçue pour que, après implantation dans le corps d'une patiente, elle se remplisse de liquide et contienne peu de tissu ou cellules. On obtient ainsi une prothèse qui forme une poche de liquide, le liquide étant produit par l'organisme de la patiente. Cette coque ne permet pas l'introduction d'un pédicule.

Le document US 5 545 217 A1 décrit une coque en textile qui comprend une masse de fibres qui ne forment pas un textile. Après implantation, la coque se remplit de fluide et l'on espère que des tissus vont s'y développer. On obtient ainsi une coque remplie de tissus et de fluide biologique générés par l'organisme de la patiente traitée.

L'article « Tissue-engineered Breast Reconstruction : Bridging the Gap toward Large-Volume Tissue Engineering in Humans » (M. W. Findlay et AI) publié en 2011 dans la revue PRSJournal (volume 128, number 6, pages 1206 à 1215), décrit une coque en polycarbonate qui comprend un fond et une portion bombée toutes deux perforées. La coque comprend une éponge en poly(L-lactide-co-glycolide) qui occupe la moitié du volume interne de la coque. Cette coque est utilisée pour la reconstruction mammaire dans une technique qui utilise un pédicule vasculaire dérivé et un tissu adipeux. Le pédicule est inséré dans la coque, le tissu adipeux est disposé sur ce dernier. Le pédicule doit permettre la vascularisation et donc l'alimentation du tissu adipeux qui doit croître et remplir la coque. On obtient ainsi un volume de tissu graisseux qui se forme *in situ* dans le corps de la patiente. Les résultats indiquent que l'éponge gêne la prolifération des cellules du tissu adipeux et finalement aucune vascularisation de ce dernier n'a pu être obtenue.

Pour tenter de remédier à ce problème, le document US 2014/0135925 A1 propose un implant mammaire qui comprend une coque en deux parties. La coque comprend un fond plat qui est équipé d'ailettes perpendiculaires au fond et une portion bombée qui comprend des cloisons internes parallèles entre elles. Lorsque la coque est formée par assemblage du fond et de la partie bombée, on obtient un espace cloisonné qui permet le dépôt d'une couche de tissu adipeux vascularisé qui remplit ainsi toute la coque. La coque comprend deux ouvertures pour le passage d'un pédicule destiné à alimenter le tissu adipeux logé dans la coque. Ce type de coque est difficile à réaliser et le dépôt du tissu dans cette dernière n'est pas aisé.

De même, le document US 2014/135925 A1 décrit toutes les techniques utilisables pour la pose d'une prothèse mammaire. La prothèse décrite dans ce document comporte une coque qui permet l'insertion d'un lit vasculaire à l'intérieur de la coque, ce lit remplissant, dès la pose de la coque, tout le volume de cette dernière. La coque comporte une seule ouverture pour l'insertion du lit vasculaire, lequel zigzague dans la coque, soutenu par des ailettes internes transversales. La coque est biorésorbable. Cette coque ne comporte pas de textile ni d'ouvertures pour le passage d'un pédicule à travers la coque.

L'article intitulé « Creation of a Large Adipose Tissue Construct in Humans Using a Tissue-engineering Chamber: A Step Forward in the Clinical Application of Soft Tissue Engineering » (W.A. Morrison et AI.) publié dans la revue EbioMedecine en Avril 2016 décrit l'utilisation d'une coque ou chambre en acrylique, perforée, pour la technique chirurgicale précédemment décrite pour la reconstruction mammaire. La coque sans fond est déposée sur un lambeau graisseux comprenant un pédicule vasculaire au niveau de l'emplacement du sein de la patiente ayant subi une mastectomie. La plaie est refermée et l'on observe le développement du tissu graisseux. Celui-ci se développe et se vascularise chez certaines patientes seulement, mais ne parvient pas à remplir la chambre. Un cas de contracture capsulaire est également relevé. Par ailleurs, la coque n'est pas résorbable et un deuxième acte chirurgical est nécessaire pour la retirer.

Un objet de la présente invention est de proposer un nouveau dispositif apte à être implanté dans le corps d'un sujet pour remplacer et/ou augmenter un volume de tissu mou.

Un autre objet de la présente invention est de proposer un nouveau dispositif apte à être implanté dans le corps d'un sujet qui permet la croissance de cellules apte à former un tissu mou, ce nouveau tissu mou formé *in situ* dans le corps permet de remplacer et/ou d'augmenter un volume de tissu mou du corps du sujet.

La présente invention concerne un dispositif apte à être implanté dans le corps d'un sujet pour former un implant apte à remplacer et/ou augmenter un volume de tissu mou, ledit dispositif étant du type comprenant une armature tridimensionnelle qui définit un espace interne à ladite armature. Selon l'invention, de manière caractéristique, ladite armature est bio résorbable, elle comprend deux ouvertures latérales formant un passage transversal permettant l'insertion d'un pédicule vasculaire et le dispositif comprend, en outre, au moins deux feuilles de textile bio résorbable aptes à être empilées l'une sur l'autre dans ledit espace interne à ladite armature.

Les Inventeurs ont en effet mis en évidence que la présence des feuilles permettait d'obtenir une masse tissulaire homogène et compacte qui pouvait être facilement insérée dans l'armature, éventuellement taillée selon la forme désirée et/ou incisée, notamment pour y insérer un pédicule vasculaire. La masse tissulaire peut, de plus, se développer *in situ* dans le corps du sujet de manière à remplir l'armature, le volume restant à remplir étant plus faible que si l'armature était vide.

Par ailleurs, l'armature rigide forme un volume qui délimite la croissance des adipocytes et qui crée une chambre de croissance cellulaire.

De préférence, les feuilles ont une forme adaptée à la section de l'armature selon laquelle elles sont empilées. Les feuilles peuvent être disposées parallèlement au fond (éventuellement ouvert) de l'armature ou perpendiculairement à ce dernier.

L'armature n'est pas limitée selon l'invention ; elle peut comprendre un fond amovible ou non, qui coopère avec le bord de l'armature ou les extrémités des arceaux définis plus loin, pour fermer cette dernière.

Selon un premier mode de réalisation, l'armature comporte une paroi qui définit un bord et une pluralité de perforations réparties sur ladite paroi et/ou une portion ouverte ménagée dans ladite paroi et s'étendant au-dessus dudit bord de ladite armature, ladite portion ouverte s'étendant depuis le sommet du dôme lorsque ladite armature a sensiblement la forme d'un dôme. La présence de perforations ou de la partie ouverte favorise la croissance *in situ* -dans le corps du sujet- des cellules de l'empilement logé dans l'armature, comme expliqué ultérieurement.

Selon une variante qui peut être combinée avec le mode de réalisation précité, ladite paroi comprend, en outre, deux ouvertures latérales formant un passage transversal au niveau dudit bord de ladite armature et/ou au-dessus dudit bord.

Les ouvertures latérales peuvent, par exemple, être des échancrures qui s'étendent depuis le bord de l'armature et sur une partie de sa hauteur. De telles ouvertures permettent d'ajuster facilement le positionnement en hauteur du pédicule lors de la pose du dispositif de l'invention dans le corps du sujet. Il est ainsi possible d'insérer le pédicule dans l'empilement de feuilles, le pédicule étant soutenu par une ou plusieurs feuilles et recouvert également d'une ou plusieurs feuilles. Une telle disposition favorise la vascularisation de la masse de tissu formée *in situ.*

L'armature peut également comporter des arceaux assemblés dont les extrémités libres sont situées dans un même plan. Les arceaux peuvent être assemblés selon une de leurs extrémités ou au niveau de leur longueur. Ce type d'armature permet d'obtenir des formes adaptées, par exemple, dans le cas d'un implant mammaire.

L'utilisation d'arceaux permet de réduire la quantité de matière formant l'armature. Celle-ci est légère, présente une grande surface en contact avec le corps du sujet lorsqu'elle est implantée dans le corps du sujet et est donc facilement résorbable par ce dernier. Elle peut également facilement être produite, par exemple, par impression 3D.

L'armature peut ainsi comporter un premier groupe d'arceaux externes et un deuxième groupe d'arceaux internes, situés dans l'espace défini par lesdits arceaux externes, les extrémités libres desdits arceaux desdits premier et deuxième groupes étant situées dans un même plan. On obtient ainsi une armature solide et rigide qui permet de bien maintenir en place les feuilles.

De préférence, ledit textile est formé d'un entrelacement d'au moins deux fils, ledit textile forme une structure tridimensionnelle qui comporte au moins deux couches de surface superposées, formant les deux faces opposées dudit textile lesdits fils relient lesdites couches de surface, lesdits fils entrelacés forment des pores qui traversent l'épaisseur dudit textile et ledit textile comporte, de préférence, des points de liaison entre lesdits fils.

Les points de liaison sont répartis de préférence de manière à ce que lesdits couches puissent s'écarter l'une de l'autre dans le sens de l'épaisseur du textile sur au moins 20%, de préférence au moins 30% et au moins 50% de la surface du textile. Des points de liaison assurent la cohésion du textile et sont répartis de manière à former dans le textile des sortes de poches au niveau desquels les couches peuvent s'écarter.

Les Inventeurs ont mis en évidence qu'un tel textile permettait une bonne accroche des cellules et permettait ainsi de former une masse compacte facile à manipuler lors de son insertion dans l'armature, au cours de la pose de l'implant. Cette masse peut être taillée, coupée pour lui donner la forme voulue ou réduire son volume ce qui facilite la pose de l'implant. L'armature peut ainsi présenter une forme simple qui permet de la fabriquer à moindre coût, par exemple, par impression 3D. Les deux couches superposées pouvant s'écarter, le textile permet de loger les cellules de manière durable et de former un réseau compact et présentant une bonne cohésion.

Avantageusement, ledit textile comporte, en outre, au moins une couche intercalaire disposée entre lesdites couches de surface, lesdits fils relient lesdites couches de surface à ladite couche intercalaire, lesdits fils entrelacés forment des jours sur ladite couche intercalaire, de préférence, lesdits jours de ladite couche intercalaire ne correspondent pas avec lesdits jours desdites faces opposées dudit textile et ledit textile comporte de préférence, des points de liaison entre lesdits fils.

Le fait que les jours des faces ne coïncident pas ou seulement partiellement, c'est-à-dire ne soient pas disposés en regard l'un de l'autre, fait que le textile forme un réseau, une sorte de filet tridimensionnel dans lequel les cellules vont s'accrocher ou par lequel elles vont être au moins retenues.

La présence de points de liaison permet de couper le textile sans qu'il ne se désagrège ou perde ses propriétés mécaniques sur une grande étendue.

La couche intercalaire améliore encore l'accroche des cellules et donc la cohésion de la masse formée. La feuille forme ainsi un réseau où se logent les cellules. Lorsque les jours des trois couches ne coïncident pas ou seulement partiellement, on obtient un réseau dense qui soutient bien la masse formée par les cellules ; lorsque celles-ci vont se diviser dans le corps du sujet, on obtient une masse dense et homogène. On observe également, dans ce cas, une croissance plus rapide des cellules, la formation d'une masse homogène car la croissance des cellules est guidée grâce au textile. Un tel textile permet la vascularisation et diminue la mortalité cellulaire. Il augmenta la cohésion de la masse obtenue, notamment de la masse graisseuse.

Le textile est également un support d'accroche pour des éléments cellulaires et les facteurs de croissance (élément protéique solubles).

Avantageusement, ledit textile comporte des pores de diamètres dispersés, la moyenne arithmétique du diamètre desdits pores est sensiblement égale ou inférieure à 3,5 et sensiblement égale ou supérieur à 1,5 et de préférence sensiblement égal à 2 et en ce que 75% desdits pores présentent un diamètre sensiblement égal ou supérieur à 2 et sensiblement égal ou inférieur à 3,5. Le diamètre des pores est lié notamment au nombre de points de liaison et à leur densité dans le textile. Les Inventeurs ont constaté qu'un tel diamètre de pore permettait d'obtenir une bonne croissance cellulaire et une masse homogène facile à implanter.

Avantageusement, ledit textile présent une déformation à la rupture dans le sens machine et/ou dans le sens travers inférieure à 50 %. On obtient ainsi une masse homogène présentant une bonne cohésion ce qui la rend facile à manipuler et à tailler, le cas échéant.

Le textile peut être formé d'au moins deux fils de diamètres différents et le rapport des diamètres des fils peut être notamment compris entre 2 et 3. Les diamètres différents permettent de former l'espace précité et de former facilement la couche intercalaire, le fil le plus fin pouvant former une couche sans toucher les couches au-dessus ou en dessous si l'écart entre ces couches est formé, par exemple par la section du fil de plus gros diamètre.

Les Inventeurs ont également mis en évidence que de bons résultats en termes de cohésion de la masse tissulaire pouvaient être obtenus, en particulier lorsque ledit textile présente une perméabilité à l'air supérieure ou égale à 10 000L/m$^2$ et une épaisseur supérieure ou égale à 0,50 mm.

Selon un mode de réalisation, qui eut être combiné avec l'une quelconque de ceux précités, le dispositif comporte un empilement d'au moins deux feuilles de textile comprenant entre elles une couche de cellules choisies parmi les adipocytes, les cellules aptes à se différencier en adipocytes et les mélanges de ces deux types de cellules et ledit empilement est logé dans ladite armature et la remplit au moins partiellement et de préférence partiellement. La présence d'un vide dans l'armature favorise la croissance des cellules situées entre les feuilles dans le corps du sujet. Les cellules précitées sont de préférence obtenues par utilisation du surnageant après centrifugation d'un amas graisseux prélevé sur le sujet lui-même ; une greffe autologue limitant en effet les réactions de rejet.

Également divulgués mais ne faisant pas partie de la présente invention est une masse apte à être implantée dans le corps d'un sujet pour remplacer et/ou augmenter un volume de tissu mou. Elle comprend au moins deux feuilles de textile superposées, ledit textile étant notamment résorbable, et entre lesdites feuilles superposées, des cellules choisies parmi les adipocytes, les cellules aptes à se différencier en adipocytes et les mélanges de ces deux types de cellules, ledit textile est formé d'un entrelacement d'au moins deux fils, ledit textile forme une structure tridimensionnelle qui comporte au moins deux couches de surface superposées, formant les deux faces opposées dudit textile lesdits fils relient lesdites couches de surface, lesdits fils entrelacés forment des pores qui traversent l'épaisseur dudit textile et ledit textile comporte, de préférence, des points de liaison entre lesdits fils.

La masse précitée peut également être utilisée pour une implantation directe dans le corps du sujet, sans armature.

Avantageusement, ledit textile comporte, en outre, au moins une couche intercalaire disposée entre lesdites couches de surface, lesdits fils relient lesdites couches de surface à ladite couche intercalaire, lesdits fils entrelacés forment des jours sur ladite couche intercalaire, de préférence, lesdits jours de ladite couche intercalaire ne correspondent pas avec lesdits jours desdites faces opposées dudit textile et ledit textile comporte de préférence points de liaison entre lesdits fils. Les mêmes avantages et caractéristiques que ceux définis en référence au dispositif sont applicables à la masse.

Avantageusement, le textile comporte des pores de diamètres dispersés, la moyenne arithmétique du diamètre desdits pores est sensiblement égale ou inférieure à 3,5 et sensiblement égale ou supérieur à 1,5 et de préférence sensiblement égal à 2 et en ce que 75% desdits pores présentent un diamètre sensiblement égal ou supérieur à 2 et sensiblement égal ou inférieur à 3,5.

Avantageusement, ledit textile présente une déformation à la rupture dans le sens machine et/ou dans le sens travers inférieure à 50 %.

Le textile est de préférence bio résorbable.

Le textile est avantageusement formé d'au moins deux fils de diamètre différent et le rapport des diamètres des fils est notamment compris entre 2 et 3.

Avantageusement, le textile présente une perméabilité à l'air supérieure ou égale à 10 000 L/m$^2$ et une épaisseur supérieure ou égale à 0,50 mm. Les avantages procurés sont les mêmes que ceux décrit en référence au dispositif de la présente invention.

La présente invention concerne également une méthode *in vitro* de fabrication d'un implant apte à être disposé dans le corps d'un sujet pour le remplacement et/ou pour l'augmentation d'un volume de tissu mou.

Selon ce procédé, de manière caractéristique,

-    on fournit une armature bio résorbable présentant une taille et une forme préalablement déterminées ; ladite armature étant tridimensionnelle (1) définissant un espace interne, étant bio résorbable, et comprenant deux ouvertures latérales formant un passage transversal permettant l'insertion d'un pédicule vasculaire;
-    on fournit au moins deux feuilles d'au moins un textile bio résorbable formé d'un entrelacement d'au moins deux fils qui forment une structure tridimensionnelle
-    on réalise au moins un premier empilement d'au moins deux desdites feuilles entre lesquelles on dépose des cellules choisies parmi les adipocytes, les cellules aptes à se différencier en adipocytes et les mélanges de ces deux types de cellules, lesdites cellules provenant de préférence dudit sujet ;
-    on dispose ledit premier empilement dans ladite armature pour l'implantation de cette dernière dans le corps d'un sujet.

Selon un mode de mise en œuvre particulier, avant ou après l'insertion dudit empilement dans ladite armature, on pratique une incision dans ledit empilement, notamment parallèlement au plan desdites feuilles, sensiblement au niveau desdites ouvertures latérales et notamment au-dessus dudit bord de ladite armature. Cette incision permet l'insertion du pédicule vasculaire.

Selon un deuxième mode de mise en œuvre, on réalise un deuxième empilement d'au moins deux desdites feuilles entre lesquelles on dépose des cellules choisies parmi les adipocytes, les cellules aptes à se différencier en adipocytes et les mélanges de ces deux types de cellules, lesdites cellules provenant de préférence dudit sujet ;

-    on dispose lesdits premier et deuxième empilements l'un sur l'autre dans ladite armature, de manière à ce que ledit passage transversal débouche sensiblement entre ledit premier et ledit deuxième empilement.

Dans ce cas, le pédicule est disposé entre les deux empilements à travers le passage transversal.

Avantageusement, on fournit un textile formé d'un entrelacement d'au moins deux fils, en ce que ledit textile forme une structure tridimensionnelle qui comporte au moins deux couches de surface superposées, formant les deux faces opposées dudit textile, lesdits fils relient lesdites couches de surface, lesdits fils entrelacés forment des pores qui traversent l'épaisseur dudit textile et ledit textile comporte, de préférence, des points de liaison entre lesdits fils.

Les avantages obtenus sont les mêmes que ceux précités en référence au dispositif.
Avantageusement, on fournit un textile qui comporte, en outre, au moins une couche intercalaire disposée entre lesdites couches de surface, lesdits fils relient lesdites couches de surface à ladite couche intercalaire, lesdits fils entrelacés forment des jours sur ladite couche intercalaire, de préférence, lesdits jours de ladite couche intercalaire ne correspondent pas avec lesdits jours desdites faces opposées dudit textile et ledit textile comporte de préférence points de liaison entre lesdits fils. Avantageusement, ledit textile comporte des pores de diamètres dispersés, la moyenne arithmétique du diamètre desdits pores est sensiblement égale ou inférieure à 3,5 et sensiblement égale ou supérieur à 1,5 et de préférence sensiblement égal à 2 et en ce que 75% desdits pores présentent un diamètre sensiblement égal ou supérieur à 2 et sensiblement égal ou inférieur à 3,5.
Le textile présente de préférence, une déformation à la rupture dans le sens machine et/ou dans le sens travers inférieure à 50 %.
Le textile est de préférence, formé d'au moins deux fils de diamètre différent et le rapport des diamètres des fils est notamment compris entre 2 et 3. Avantageusement, ledit textile présente une perméabilité à l'air supérieure ou égale à 10 000 L/m$^2$ et une épaisseur supérieure ou égale à 0,50 mm.
La présente invention concerne également un implant mammaire comportant le dispositif et/ou la masse selon l'invention.

**[Définitions]**

Dans toute la présente demande, les termes «apte à être implanté dans le corps d'un sujet» indiquent que toutes les parties du dispositif qui sont destinées à demeurer un certain temps dans le corps du sujet sont en matériau(x) adapté(s), c'est-à-dire en matériau biocompatible et éventuellement bio résorbable.

Les termes « tissu mou » désignent, au sens de la présente invention, les tissus adipeux et plus particulièrement le tissu adipeux profond.

Le terme « fil » désigne un mono filament ou fibre ou un groupe de mono filaments ou fibres éventuellement torsadés.

Le terme «point de liaison » désigne un nœud formé par un enlacement serré d'un fil sur lui-même ou de deux fils l'un sur l'autre. Un nœud est tel que même si la tension des fils est nulle, les fils ne se séparent pas l'un de l'autre. Le terme « point de liaison » désigne également toute zone ponctuelle de fixation. de deux portions d'un même fil ou de deux portions de deux fils distincts l'une sur l'autre ou l'une avec l'autre. Il peut s'agir, par exemple, d'une zone ponctuelle créée par collage ou par fusion du matériau des portions de fil(s).

Le terme « entrelacement » désigne un entrecroisement d'un fil sur lui-même ou de deux fils entre eux ; lorsque la tension entre deux fils entrelacés est nulle, l'entrelacement se défait.

Le terme « jours » désignent des vides délimités par au moins un fil et formés dans une couche d'un entrelacement de fil(s). Les jours sont formés par l'écartement de deux portions d'un même fil ou d'au moins deux fils.

Le terme « pores » désigne des vides formés dans l'épaisseur du textile, qui traversent l'épaisseur du textile et débouchent sur les deux faces de ce dernier ; les pores sont formés par l'écartement des fils entrelacés.

Les termes « perméabilité à l'air » font référence à la capacité du textile à laisser s'écouler l'air au travers de sa structure fibreuse.

Le terme « épaisseur » fait référence à la valeur de l'épaisseur obtenue par calcul à partir du grammage du textile.

Le terme « bio résorbable » désigne un objet qui a la propriété de se dégradé dans le corps d'un être vivant, les produits de dégradation étant évacués par ledit organisme vivant de manière à ce que ledit objet disparaisse au bout d'un temps plus ou moins long qui peut être grossièrement déterminé et qui est fonction du matériau dudit objet.

Le terme « porosité » représente le rapport volume de vide / volume total d'un textile; la porosité correspond à la formule 1-$\alpha$, où $\alpha$ est la compacité et se calcule en fonction de l'épaisseur mesurée et du grammage selon la formule suivante : $\alpha = \dfrac{G}{\rho f \times Z}$ dans laquelle G est le grammage mesuré en g/m$^2$, $\rho f$ la masse volumique du matériau des fils kg/m$^3$ et Z l'épaisseur mesurée en mm.

Les termes « cellules aptes à se différencier en cellules adipeuses » font référence aux cellules souches adultes, notamment aux cellules mésenchymateuses adultes (provenant d'un individu adulte) qui sont aptes à se différencier en cellules pouvant se différencier en adipocytes.

Les termes « sens machine » désignent le sens dans lequel le textile est fabriqué.

Les termes « sens travers » désignent le sens perpendiculaire à la direction de fabrication du textile.

Les termes « module d'élasticité » désignent le module d'Young.


**[FIGURES]**

La présente invention, ses caractéristiques et les divers avantages qu'elle procure apparaîtront mieux et seront mieux compris à la lecture de la description qui suit de trois modes de réalisation particuliers de la présente invention, présentés à titre d'exemples d'illustration non limitatifs qui fait référence aux dessins sur lesquels :

- les Fig. 1a et 1b représentent de manière schématique des vues en perspective de deux modes de réalisation de l'armature du dispositif de l'invention ;
- la Fig. 2 représente une vue schématique d'une coupe transversale d'un troisième mode de réalisation particulier du dispositif de l'invention prêt pour son implantation dans le corps d'un sujet ;
- les Fig. 3A à 3C représentent des photographies au microscope optique des différents textiles utilisés pour la fabrication des feuilles équipant le dispositif de l'invention et référencés de A à C ;
- la Fig. 4 représente une photographie des masses tissulaires obtenues respectivement avec des feuilles de textile A, à gauche et à droite avec des feuilles de textile B ;
- la Fig. 5 représente une photographie montrant à gauche une masse tissulaire obtenue avec le textile A dans un tube de solution aqueuse et à droite, un masse tissulaire obtenue avec le textile B également plongée dans un tube contenant une solution aqueuse ;
- la Fig. 6 représente une photographie montrant l'aspect de la masse obtenue avec des disques de textile A (à gauche) et celui de la masse obtenue avec des disques de textile B (à droite), après 24h dans du sérum physiologie ;
- la Fig. 7 représente une photographie montrant une coupe transversale de la masse obtenue avec des disques de textile A, réalisée au bistouri et, une coupe transversale de la masse tissulaire obtenue avec des disques de textile B ;
- la Fig. 8 représente une photographie de l'aspect de chacune des masses tissulaires après arrachement d'une feuille, la photographie du haut concerne la masse obtenue avec des feuilles de textile A et celle du bas, la masse obtenue avec des feuilles de textile B ;
- la Fig. 9 représente une photographie obtenue au microscope optique d'une feuille de textile A (en haut) et d'une feuille de textile B sur lesquelles on a déposé du surnageant comme ultérieurement expliqué ;
- la Fig. 10 ne représente pas un mode de réalisation de l'armature du dispositif de l'invention ; et
- la Fig. 11 représente des diagrammes de dispersion de la taille des pores pour chacun des échantillons A à C.

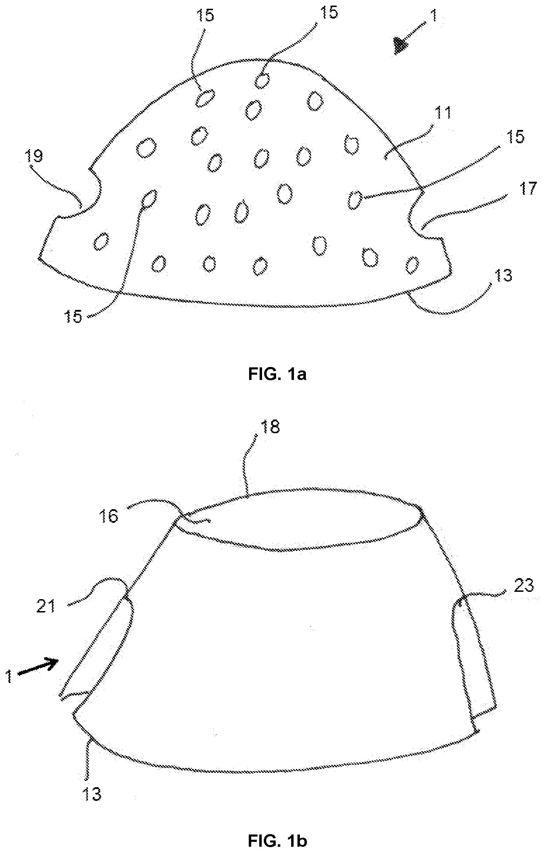

En référence à la Fig. 1a, un premier mode de réalisation de l'armature du dispositif de l'invention va être décrit. L'armature 1 comporte une paroi 11 qui définit un bord inférieur 13. La paroi 11 comporte une pluralité de perforations 15 et deux ouvertures latérales 17 et 19 qui perforent la paroi 11 et sont situées sensiblement l'une en regard de l'autre de manière à définir un passage transversal à l'armature 1. Les ouvertures 17 et 19 sont situées au-dessus du bord inférieur 13.

Le deuxième mode de réalisation va maintenant être décrit en référence à la Fig. 1b ; les éléments en commun avec le premier mode de réalisation sont référencés à l'identique. La paroi 11 de l'armature comporte une portion ouverte 16 située au-dessus du fond ouvert de l'armature 1. Cette portion ouverte forme un bord supérieur 18. La paroi comporte également deux échancrures 21 et 23 qui coupent le bord inférieur 13 et forment des ouvertures dans la paroi 11 au-dessus du bord inférieur 13. Ces deux échancrures 21 et 23 forment un passage qui traverse de part-en-part l'armature 1.

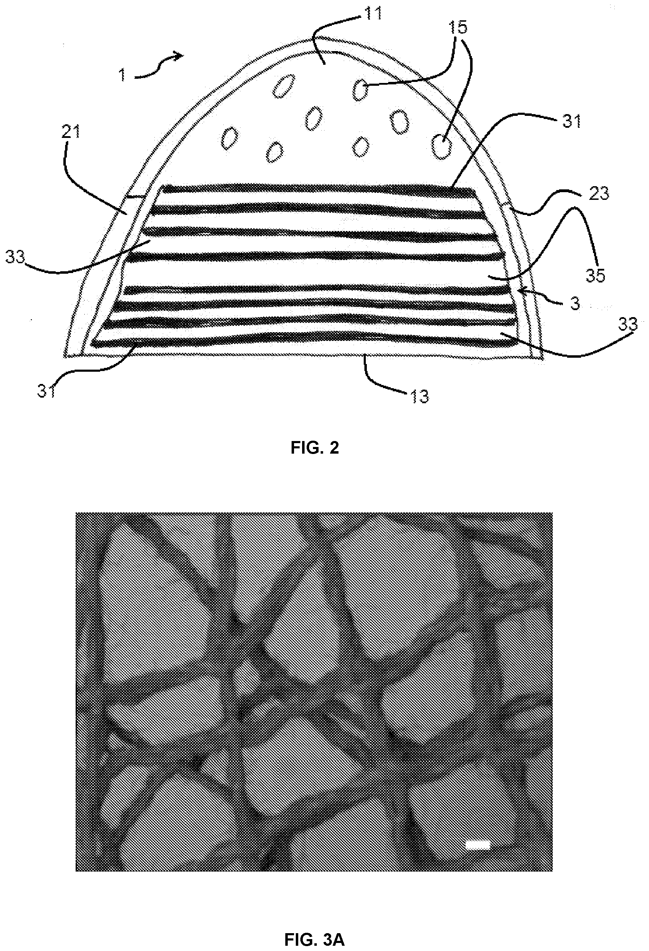

Un troisième mode de réalisation du dispositif de l'invention va maintenant être décrit en référence à la Fig. 2. Les éléments en commun avec les deux modes de réalisation précités sont référencés à l'identique.

L'armature 1 comporte une paroi 11 qui présente une pluralité de perforations 15 et deux échancrures 21 et 23. L'armature comprend un empilement 3 qui est formé de feuilles de textiles 31 (en noir) et de couches d'adipocytes 33 (en blanc) ou tissu graisseux superposées. L'empilement 3 comprend un passage traversant 35 ménagé par incision par exemple. Les ouvertures de ce passage 35 correspondent aux échancrures 21 et 23. Il est ainsi possible en introduisant un outil adapté à travers une des échancrures 21 et 23 et en l'accrochant à un pédicule vasculaire, d'introduire ce pédicule dans le passage 35 et de le déposer, par exemple, sensiblement au centre de ce dernier.

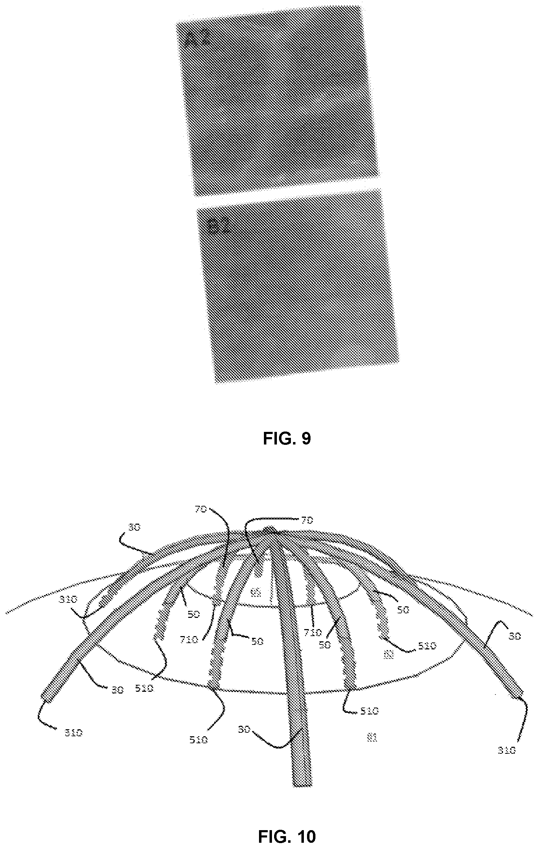

Une autre armature va maintenant être décrite en référence à la Fig. 10.

Dans ce mode de réalisation particulier, l'armature 1 comporte trois séries d'arceaux 30, 50 et 70. Les arceaux de la première série 30 forment 6 pieds qui correspondent aux extrémités 310 des arceaux et qui reposent sur une première feuille textile 61. Ces arceaux forment un premier dôme. A l'intérieur de ce dôme, se trouve la deuxième série d'arceaux 50 qui comporte 6 pieds 510 qui correspondent aux extrémités libres des arceaux et qui reposent sur la première feuille 61. Les arceaux de la deuxième série 50 traversent une deuxième feuille 63 qui est disposée au-dessus de la première feuille 61. La deuxième feuille 63 a une surface plus réduite que la première feuille 61. Les arceaux de la deuxième série 50 traversent la deuxième feuille 63 soit au niveau de ses pores, soit au niveau d'ouvertures ménagées cette dernière. La troisième série d'arceaux 70 est située à l'intérieur du volume délimité par les arceaux 5. Les arceaux 70 comportent 6 pieds 710 qui correspondent aux extrémités libres des arceaux 70 et qui reposent sur la première feuille 61. Les arceaux 70 traversent une troisième feuille 65 de surface inférieure à celle de la première et de la deuxième feuille. Un telle armature permet avec un minimum de matière de définir une chambre qui permet de bloquer l'empilement des feuilles et favorise la croissance cellulaire lorsque des cellules sont déposées sur les feuilles. Elle est donc facilement résorbable dans l'organisme d'un sujet et permet de former facilement l'empilement par simple perçage des feuilles avec les arceaux 30, 50 ou 70. Les arceaux définissent naturellement des ouvertures qui forment un passage traversant permettant l'insertion du pédicule dans la structure tridimensionnelle.

## [EXEMPLES]

Plusieurs textiles ont été étudiés en référence à la formation de l'empilement qui forme également la masse tissulaire de l'invention.

## Caractérisation des textiles

Trois échantillons de textiles différents, référencés de A à C, ont été étudiés. Tous ces textiles sont obtenus chacun par entrelacement de deux fils. Tous les textiles comportent des points de liaison qui sont dans le cas présent des nœuds. Pour chacun des échantillons, on a mesuré le grammage, l'épaisseur, la perméabilité à l'air et le diamètre des fils. La porosité a été calculée comme précité.

La mesure du grammage ($g/m^2$) a été mise en œuvre selon la norme ISO 3374. Trois échantillons de chaque échantillon de textile référencé A à C sont prélevés avec un emporte-pièce circulaire de 14 cm de diamètre ($100 \ cm^2$) afin d'obtenir une bonne représentativité des différents textiles. On pèse chacun des échantillons et l'on calcule le grammage en divisant la masse mesurée par la surface de l'échantillon. On calcule ensuite la moyenne et l'écart-type des grammages obtenus. On retire tous les échantillons dont le grammage n'est pas compris dans la plage [moyenne - écart type ; moyenne + écart type] et on recommence à prélever des échantillons de textile jusqu'à obtenir cinq échantillons par textile de référence compris dans la plage de grammage précitée. La pesée est effectuée avec une balance Sartorius ENTRIS224i-1S.

La perméabilité à l'air est mesurée à l'aide d'un perméabilimètre à air AP-36 de VVC avec une perte de charge d'aspiration fixée à 196 Pa selon la norme AFNOR G07-111. Le résultat de perméabilité à l'air est exprimé en L/m$^2$/s ce qui représente un débit d'air par rapport à une surface de 1 m$^2$. Les échantillons de textile utilisés pour la mesure de la perméabilité ont une surface de 20 cm$^2$.

La mesure de l'épaisseur est réalisée selon la norme EN ISO 5084 sur un appareil VVC 2000 avec un poids utilisé correspondant à une charge de 1 kPa, l'unité de mesure est le millimètre. Une fois que le grammage est mesuré et validé, on utilise ces mêmes échantillons pour la mesure de l'épaisseur de chacun des textiles.

La mesure du diamètre des fils est mise en œuvre avec un microscope optique. On réalise une coupe transversale du fil à l'aide d'une lame de rasoir puis on observe et mesure le diamètre des mono-filaments ou du mono filament formant le fil à l'aide d'un microscope optique binoculaire (Axiolab Pol de Carl Zeiss).

Le Tableau I suivant regroupe les différents paramètres de chacun des textiles.

Tableau I

|  | A | B | C |
|---|---|---|---|
| Grammage (g/m$^2$) | 84,41 | 129,00 | 108,78 |
| Epaisseur (mm) | 0,84 | 0,94 | 0,77 |
| Porosité (%) | 91,31 | 88,17% | 87,88 |
| Diamètre fils ($\mu$m) | 80,07 | 119,52 | 81,75 |
| Perméabilité à l'air (L/m$^2$/s) | 10960,00 | 9042,00 | 8702,00 |

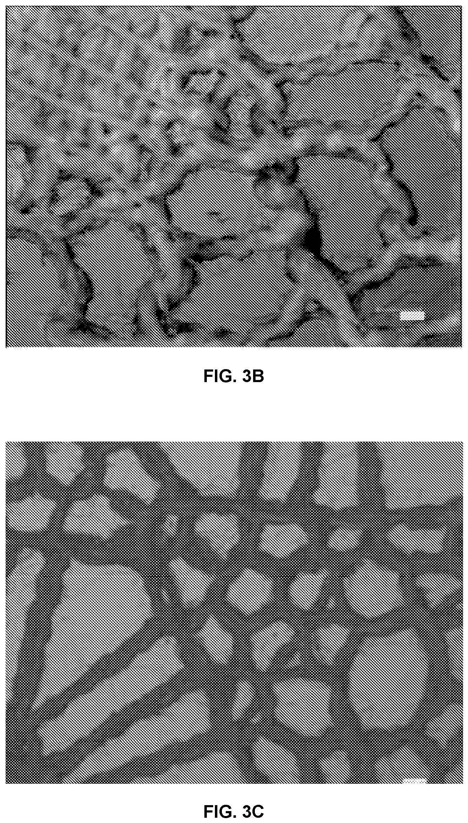

Par ailleurs, des photographies au microscope de chacun des échantillons ont également été réalisées. Ces photographies sont visibles sur les Fig. 3A à 3C.

En référence aux Fig. 3A à 3C, on remarque que l'échantillon A présente trois couches superposées de fils, les couches ne s'interpénètrent pas. L'échantillon A est obtenu par entrelacement de deux fils de diamètre différents, en l'occurrence un fil de 49 dtx avec un fil de 120 dtx. L'échantillon A comporte des jours de tailles différentes et aucune zone compacte formée par des fils côte-à-côte ayant pour conséquence de créer une zone sans jour. Les jours d'une couche ne correspondent pas à ceux des deux autres mais le textile comporte des pores qui sont formés par les zones où les jours des trois couches coïncident. On observe également que les nœuds sont suffisamment éloignés les uns des autres pour permettre localement aux couches de fils de s'écarter l'une de l'autre dans le sens de l'épaisseur du textile.

L'échantillon B ne comporte qu'une épaisseur formée d'un entrelacement de deux fils de diamètres différents. Par ailleurs, l'échantillon B présente une zone localisée plus dense qui ne comporte pas ou seulement de très petits jours (voir en haut à gauche de la photographie B).

L'échantillon C présentent deux couches de fils de diamètres différents. La taille des jours n'est pas homogène. L'échantillon C est formé d'un entrelacement de deux fils de diamètres différents. La majorité des pores est formée par des jours des deux couches qui coïncident. Les deux couches sont proches l'une de l'autre du fait d'un grand nombre de noeuds ; localement il semble qu'il n'y ait d'une couche de fils (voir la zone à gauche où les pores sont de plus grande taille). L'échantillon C comporte une zone où les fils entrelacés forment une surface de fils continue sans pores (voir en bas à gauche de la Fig. 3C).

**Etude des propriétés mécaniques des textiles**

Le Tableau II ci-dessous regroupe les résultats obtenus au cours d'essais de traction sur les textiles A à C précités.

Tableau II

| Echantillon | A_Machine | A_Travers | B | C_Machine | C_Travers |
|---|---|---|---|---|---|
| **Nombre d'éprouvettes pris en compte** | 4/5 | 3/4 | 2/4 | 4/5 | 5/5 |
| **Déformation au maximum (%)** | 32,04 | 57,13 | 67,85 | 53,31 | 91,08 |
| **Déformation à la rupture (%)** | 32,68 | 57,54 | 70,62 | 53,56 | 93,72 |
| **Force maximale (N)** | 70,53 | 153,67 | 187,60 | 87,53 | 233,58 |
| **Force à rupture (N)** | 66,65 | 143,20 | 174,50 | 83,60 | 216,98 |

(suite)

| Echantillon | A_Machine | A_Travers | B | C_Machine | C_Travers |
|---|---|---|---|---|---|
| **Contrainte maximale (Mpa)** | 0,71 | 1,54 | 1,88 | 0,88 | 2,34 |
| **Module d'élasticité (Mpa)** | 5,87 | 6,72 | 5,92 | 2,87 | 4,77 |

Les termes « déformation au maximum » désignent la déformation maximale obtenue avant la rupture. Le terme « machine » indique un essai en traction dans le sens machine. On remarque au vu des résultats du Tableau II que l'échantillon A, en sens machine, possède les valeurs les plus faibles en termes de déformation, de force maximale et force à la rupture. L'échantillon A, en sens machine, est le moins extensible de tous avec seulement 32% de déformation à la rupture et possède un module d'élasticité parmi les plus élevés avec 5,87 Mpa. L'échantillon A, en sens travers, s'allonge approximativement deux fois plus qu'en sens machine avec 57% d'allongement et possède le module d'élasticité le plus élevé. Globalement, l'échantillon A est le moins extensible de tous les échantillons tout en ayant le module d'élasticité le plus important.

L'échantillon B présente des caractéristiques mécaniques intermédiaires entre les celles des échantillons A et C ; il plus extensible que l'échantillon A dans les deux sens et moins extensible que l'échantillon C en sens travers. L'échantillon B possède un module d'élasticité proche de celui de l'échantillon A avec 5,92 Mpa.

L'échantillon C, en sens travers, est le plus extensible avec 93% en déformation à la rupture et des valeurs les plus élevées pour la force maximale et la force à la rupture mais son module d'élasticité n'atteint pas la valeur de celui de l'échantillon A avec seulement 4,77 Mpa.

**Etude de la taille des pores des échantillons**

On a utilisé un profilomètre qui permet de déterminer la rugosité et la micro géométrie d'une surface.

Le profilomètre employé est l'AltiSurf 500 provenant du fabricant Altimet.

Les mesures réalisées ne suivent pas de normes et ont un but purement qualitatif. Les dimensions des échantillons sont décrites dans le tableau III ci-dessous.

Tableau III

| Echantillon | A | B | C |
|---|---|---|---|
| **Dimensions de l'échantillon (mm × mm)** | 50 x 50 | 40 x 40 | 23 x 23 |
| **Surface (mm$^2$)** | 2500 | 1600 | 529 |

A l'aide des images obtenues par utilisation du profilomètre, on a étudié la distribution de la taille des pores grâce au logiciel ImageJ. Ce logiciel est un programme d'analyse d'image développé par le National Institute of Mental Health, Bethesda, Maryland, aux Etats-Unis. Le logiciel permet de calculer l'aire en fonction des pixels et de l'échelle imposé par l'utilisateur. Les images obtenues grâce au profilomètre sont transformées en image 8-bit, en nuances de gris. Ensuite, on utilise la fonction « Binary » afin de transformer l'image en nuances de gris en une image en noir et blanc. Une fois cette étape réalisée, on passe à l'étape de délimitation avec la fonction « Threshold » qui permet de segmenter les pores et le fond. Enfin, il est possible d'utiliser la fonction « Analyze Particles » afin de quantifier la quantité de pores ainsi que l'aire respectif de chacun de ces pores.

On peut choisir la taille des pores qui est prise en comptes dans l'analyse. Toutes les tailles ont été prises en compte. Les pores qui sont situés sur les bords de l'échantillon et qui ne sont donc pas délimités n'ont pas été pris en compte, leur taille étant indéterminable.

On a considéré pour le calcul de l'aire des pores que ces pores étaient des cercles puis on a calculé le diamètre correspondant

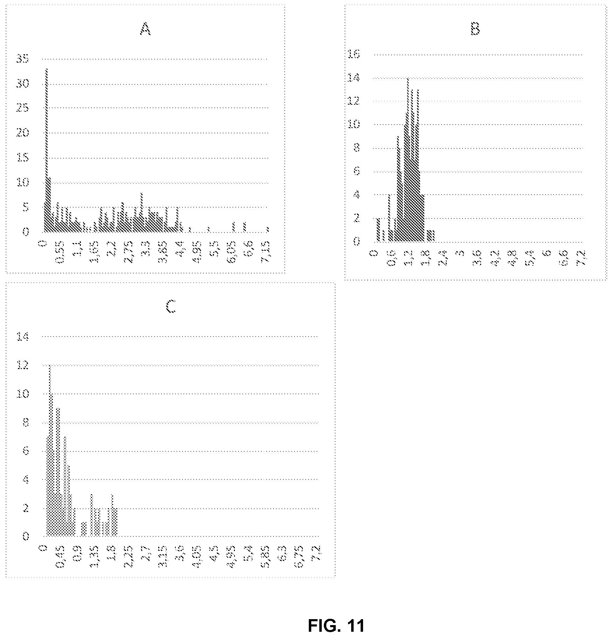

La Fig. 11 représente des diagrammes de distribution de la taille des pores. On a considéré un intervalle de 0,05 mm afin d'avoir une distribution assez fine.

Sur la base des résultats représentés sur la Fig. 11, on constate que l'échantillon A présente une distribution des diamètres de pores très hétérogène. Cette distribution définit l'intervalle sur lequel les échantillons vont être comparés. En effet, les diamètres de pores varient de 0,087 à 7,225 mm. On remarque que les pores de petites tailles sont très nombreux par rapport aux autres échantillons. On compte pour l'échantillon A jusqu'à 33 pores ayant un diamètre compris entre 0,10 et 0,15.

L'échantillon B présente une courbe de répartition de la taille des pores qui est approximativement une courbe de Gauss ; Pour l'échantillon C, on observe un nombre plus élevé de pores de petit diamètre. Le Tableau IV qui suit regroupe les différentes valeurs des différentes moyennes des tailles de pores pour chacun des échantillons.

Tableau IV

| Echantillons | A | B | C |
|---|---|---|---|
| **Moyenne Arithmétique (mm)** | 2,002 | 1,211 | 0,629 |
| **Moyenne Géométrique (mm)** | 1,129 | 1,135 | 0,453 |
| **Ecart-type (mm)** | 1,561 | 0,347 | 0,531 |
| **Variance (mm$^2$)** | 2,436 | 0,121 | 0,282 |
| **Maximum (mm)** | 7,225 | 2,071 | 1,939 |
| **Minimum (mm)** | 0,087 | 0,118 | 0,107 |
| **Effectif** | 277 | 171 | 102 |
| **Mediane (mm)** | 2,068 | 1,223 | 0,424 |
| **Quartile 1 (mm)** | 0,407 | 1,021 | 0,242 |
| **Quartile 2 (mm)** | 2,068 | 1,223 | 0,424 |
| **Quartile 3 (mm)** | 3,233 | 1,470 | 0,742 |

La différence entre la moyenne arithmétique et géométrique en référence à l'échantillon A par rapport aux autres échantillons est significative de l'hétérogénéité de la distribution des diamètres des pores. On observe également l'écart-type le plus élevé pour l'échantillon A parmi les quatre échantillons ce qui qui montre la grande dispersion des diamètres des pores pour l'échantillon A. L'écart-type pour les échantillons B et C étant plus faible, on peut conclure que les valeurs sont regroupées autour de la moyenne arithmétique. Enfin les quartiles nous indiquent la valeur du diamètre comprenant respectivement 25, 50 et 75% de l'effectif.

**Réalisation de la masse tissulaire**

On prépare le tissu graisseux suivant les protocoles utilisés pour une autogreffe. Après lipoaspiration, le tissu graisseux est centrifugé et l'on ne récupère que la phase lipidique qui forme le surnageant, les éléments sanguins restant au fond du tube de centrifugation. La phase lipidique contient des adipocytes.

On prélève plusieurs feuilles de chacun des textiles référencés A à E. On découpe des disques de 4cm de diamètre dans chacun des textiles et on réalise un empilement de ces disques. Sur une face de chaque disque, on dispose des cellules du surnageant précité avec une pipette de 25mL. On étale le surnageant afin d'obtenir une épaisseur constante d'adipocytes sur chaque disque. On obtient des empilements formés de disques de textile espacés d'une couche d'adipocytes. On emballe ensuite chaque empilement dans un film de type Parafilm M® et on place le tout-pendant 24 heures à 37°C On retire ensuite le film et on examine l'aspect de la masse tissulaire ainsi formée.

**Etude des propriétés de la masse tissulaire**

On a réalisé des masses tissulaires comme précédemment expliqué avec les différents échantillons de textile. On a ensuite comparé certaines propriétés des masses tissulaires obtenues. Les résultats suivants concernent les masses obtenues respectivement avec le textile A et le textile B. Le textile C permet d'obtenir les mêmes résultats que le textile B.

a) *Aspect extérieur de la masse tissulaire*

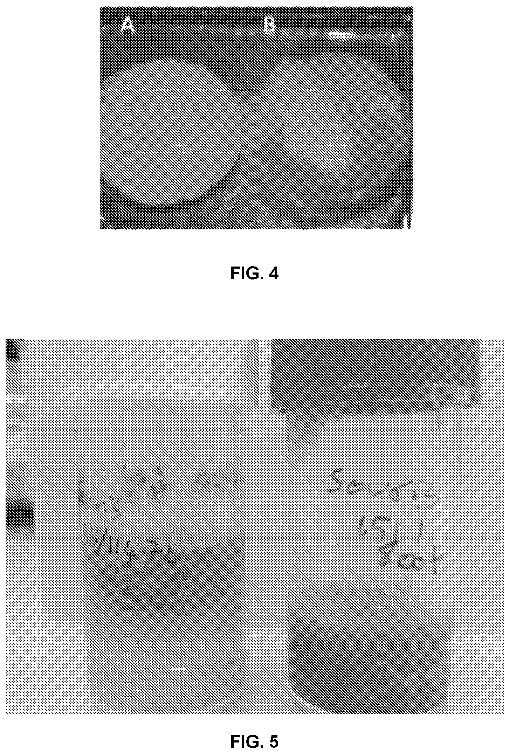

La Fig. 4 représente les masses tissulaires A et B obtenues selon le protocole précité avec respectivement des disques de textile A et des disques de textile B. Sur la Fig. 4, on constate que les disques de textile B ne permettent pas la tenue de la masse lorsque l'on retire le film. On constate que la masse B présente un étalement de son diamètre d'environ 25% ce qui correspond à un affaissement périphérique de la structure en couche formée. Au contraire, la masse tissulaire A, obtenue avec le textile A, conserve sa forme et ne s'étale pas.

*b) Cohésion dans un milieu aqueux (sérum physiologique 9 :100 NaCl : eau)*

On plonge une masse tissulaire de plus petite taille dans du sérum physiologique (9 : 100) pour évaluer sa cohésion. La Fig. 5 montre, dans le tube de gauche, une masse tissulaire A plongée dans de l'eau et dans le tube de droite, une masse tissulaire B plongée dans de l'eau. On constate que la masse A reste en un seul morceau qui flotte à la surface de l'eau tandis que pour la masse B, cette dernière s'est totalement désagrégée et forme une suspension dans l'eau.

*c) Etude du maintien de la cohésion dans le temps*

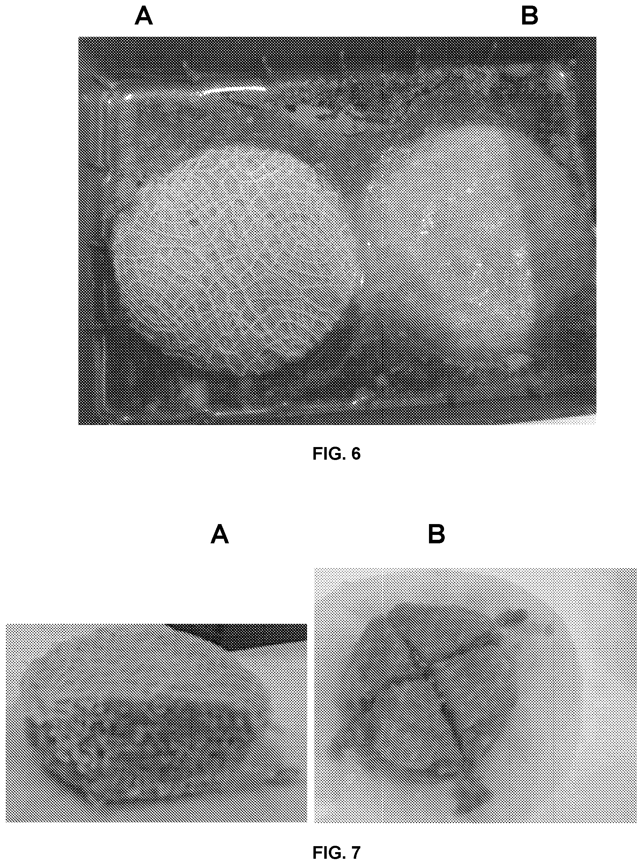

La Fig. 6 représente l'aspect de la masse A obtenue avec des disques de textile A et celui de la masse B obtenue avec des disques de textile B, après 24h ; les deux masses sont placées dans du sérum physiologique. On constate sur la Fig. 6, que la masse A reste homogène malgré l'assèchement de sa surface dû à l'évaporation du milieu. La masse B se désagrège et l'on retrouve des cellules adipeuses du surnageant dans le milieu.

*d) Dissection de la* masse *tissulaire*

La Fig. 7 montre à droite une coupe transversale de la masse A réalisée au bistouri et, à gauche une coupe transversale de la masse tissulaire B. On constate que la structure de la masse A est homogène ce qui permet d'obtenir des tranches nettes. La masse B se coupe difficilement et libère des amas graisseux qui tombent de la masse tissulaire.

*e) Test d'arrachement*

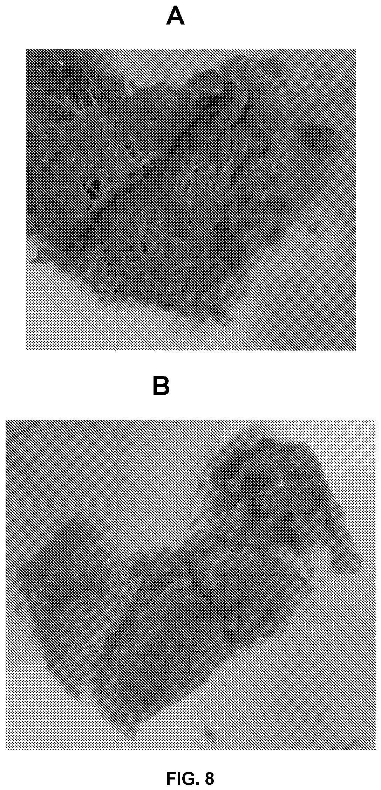

Le principe consiste à arracher un disque de chaque masse tissulaire A et B et d'observer si les cellules adipeuses restent ou non sur le disque de textile arraché. Les résultats sont visibles sur la Fig. 8. Pour la masse A, on observe que le disque de textile est entièrement recouvert de cellules adipeuses alors que pour la masse B, le disque de textile se retire facilement et il ne reste aucune cellule accrochée à sa surface.

*f) Observation microscopique*

La Fig. 9 représente des photographies obtenues au microscope optique (indiquer le grossissement et le type de microscope utilisé) d'une feuille de textile A (en haut) et d'une feuille de textile B sur lesquelles on a déposé du surnageant précité (pendant plus de 24 heures. On constate que le surnageant se réparti uniformément dans toute la structure du textile pour le textile A alors qu'il existe des zones sans surnageant pour le textile B (zones plus pâles, en bas à droite de la photographie B2).

Les résultats précités indiquent que des feuilles de textile A permettent d'obtenir une masse tissulaire homogène, compact, pouvant être découpée afin de lui conférer une forme souhaitée, par exemple. Cette masse étant compacte, elle peut également être incisée afin, notamment d'y introduire un pédicule vasculaire sans décohésion ou altération de la masse.

**Revendications**

1. Dispositif apte à être implanté dans le corps d'un sujet pour former un implant apte à remplacer et/ou augmenter un volume de tissu mou, ledit dispositif étant du type comprenant une armature tridimensionnelle (1) qui définit un espace interne à ladite armature (1), **caractérisé en ce que** ladite armature (1) est bio résorbable, **en ce qu'**elle comprend deux ouvertures latérales formant un passage transversal permettant l'insertion d'un pédicule vasculaire et **en ce qu'**il comprend, en outre, au moins deux feuilles (31 ; 61) de textile bio résorbable aptes à être empilées l'une sur l'autre dans ledit espace interne à ladite armature (1).

2. Dispositif selon la revendication 1, **caractérisé en ce que** ladite armature (1) comporte une paroi qui définit un bord (13) et une pluralité de perforations (15) réparties sur ladite paroi et/ou une portion ouverte ménagée dans ladite paroi et s'étendant au-dessus dudit bord (13) de ladite armature, ladite portion ouverte (16) s'étendant depuis le sommet du dôme lorsque ladite armature (1) a sensiblement la forme d'un dôme.

3. Dispositif selon la revendication 1, **caractérisé en ce que** ladite armature (1) comporte des arceaux (3, 5) assemblés dont les extrémités libres sont situées dans un même plan et de préférence au moins un premier groupe d'arceaux externes (3) et un deuxième groupe d'arceaux internes (5), situés dans l'espace défini par lesdits arceaux externes (3), les extrémités libres desdits arceaux desdits premier et deuxième groupes étant situées dans un même plan.

4. Dispositif selon l'une quelconque des revendications précédentes, **caractérisé en ce que** ledit textile est formé d'un entrelacement d'au moins deux fils, **en ce que** ledit textile forme une structure tridimensionnelle qui comporte au moins deux couches de surface superposées, formant les deux faces opposées dudit textile, **en ce que** lesdits fils relient lesdites couches de surface, **en ce que** lesdits fils entrelacés forment des pores qui traversent l'épaisseur dudit textile et **en ce que** ledit textile comporte, de préférence, des points de liaison entre lesdits fils.

5. Dispositif selon la revendication 4, **caractérisé en ce que** ledit textile. comporte, en outre, au moins une couche intercalaire disposée entre lesdites couches de surface, **en ce que** lesdits fils relient lesdites couches de surface à ladite couche intercalaire, **en ce que** lesdits fils entrelacés forment des jours sur ladite couche intercalaire, **en ce que**, de préférence, lesdits jours de ladite couche intercalaire ne correspondent pas avec lesdits jours desdites faces opposées dudit textile et **en ce que** ledit textile comporte de préférence des points de liaison entre lesdits fils.

6. Dispositif selon l'une quelconque des revendications précédentes, **caractérisé en ce que** ledit textile comporte des pores de diamètres dispersés, la moyenne arithmétique du diamètre desdits pores est sensiblement égale ou inférieure à 3,5 et sensiblement égale ou supérieur à 1,5 et de préférence sensiblement égal à 2 et **en ce que** 75% desdits pores présentent un diamètre sensiblement égal ou supérieur à 2 et sensiblement égal ou inférieur à 3,5.

7. Dispositif selon l'une quelconque des revendications précédentes, **caractérisé en ce que** ledit textile présent une déformation à la rupture dans le sens machine et/ou dans le sens travers inférieure à 50 %.

8. Dispositif selon l'une quelconque des revendications précédentes, **caractérisé en ce que** ledit textile est formé d'au moins deux fils de diamètre différent et **en ce que** le rapport des diamètres des fils est notamment compris entre 2 et 3.

9. Dispositif selon l'une quelconque des revendications précédentes, **caractérisé en ce que** ledit textile présente une perméabilité à l'air supérieure ou égale à 10 000 L/m$^2$ et une épaisseur supérieure ou égale à 0,50 mm.

10. Dispositif selon l'une quelconque des revendications précédentes, **caractérisé en ce qu'**il comporte un empilement (3) d'au moins deux feuilles de textile (31 ; 61) comprenant entre elles une couche de cellules (33) choisies parmi les adipocytes, les cellules aptes à se différencier en adipocytes et les mélanges de ces deux types de cellules et **en ce que** ledit empilement est logé dans ladite armature et la remplit au moins partiellement et de préférence partiellement.

11. Méthode *in vitro* de fabrication d'un implant apte à être disposé dans le corps d'un sujet pour le remplacement et/ou pour l'augmentation d'un volume de tissu mou, **caractérisé en ce que** :

    - on fournit une armature (1) présentant une taille et une forme préalablement déterminées; ladite armature étant tridimensionnelle (1) définissant un espace interne, étant bio résorbable, et comprenant deux ouvertures latérales formant un passage transversal permettant l'insertion d'un pédicule vasculaire;
    - on fournit au moins deux feuilles (31) d'au moins un textile formé d'un entrelacement d'au moins deux fils qui forment une structure tridimensionnelle
    - on réalise au moins un premier empilement (3) d'au moins deux desdites feuilles entre lesquelles on dépose des cellules choisies parmi les adipocytes, les cellules aptes à se différencier en adipocytes et les mélanges de ces deux types de cellules, lesdites cellules provenant de préférence dudit sujet ;
    - on dispose ledit premier empilement (3) dans ladite armature (1) pour l'implantation de cette dernière dans le corps d'un sujet.

12. Méthode selon la revendication 11, **caractérisée en ce que** :

    - avant ou après l'insertion dudit empilement (3) dans ladite armature (1), on pratique une incision dans ledit empilement (3), notamment parallèlement au plan desdites feuilles (31), sensiblement au niveau desdites ouvertures latérales et notamment au-dessus dudit bord de ladite armature (1).

13. Méthode selon la revendication 11 ou 12, **caractérisée en ce que**

    - on réalise un deuxième empilement d'au moins deux desdites feuilles entre lesquelles on dépose des cellules choisies parmi les adipocytes, les cellules aptes à se différencier en adipocytes et les mélanges de ces deux types de cellules, lesdites cellules provenant de préférence dudit sujet ;

- on dispose lesdits premier et deuxième empilements l'un sur l'autre dans ladite armature, de manière à ce que ledit passage transversal débouche sensiblement entre ledit premier et ledit deuxième empilement.

14. Méthode selon l'une quelconque des revendications précédentes, **caractérisée en ce qu'**on fournit un textile formé d'un entrelacement d'au moins deux fils, **en ce que** ledit textile forme une structure tridimensionnelle qui comporte au moins deux couches de surface superposées, formant les deux faces opposées dudit textile **en ce que** lesdits fils relient lesdites couches de surface, **en ce que** lesdits fils entrelacés forment des pores qui traversent l'épaisseur dudit textile et **en ce que** ledit textile comporte, de préférence, des points de liaison entre lesdits fils.

15. Méthode selon l'une quelconque des revendications précédentes, **caractérisée en ce que** ledit textile comporte, en outre, au moins une couche intercalaire disposée entre lesdites couches de surface, **en ce que** lesdits fils relient lesdites couches de surface à ladite couche intercalaire, **en ce que** lesdits fils entrelacés forment des jours sur ladite couche intercalaire, **en ce que**, de préférence, lesdits jours de ladite couche intercalaire ne correspondent pas avec lesdits jours desdites faces opposées dudit textile et **en ce que** ledit textile comporte de préférence points de liaison entre lesdits fils.

**Patentansprüche**

1. Vorrichtung, die zur Implantation in den Körper einer Person imstande ist, um ein Implantat zu bilden, das imstande ist, ein Weichgewebevolumen zu ersetzen und/oder zu vergrößern, wobei die Vorrichtung des Typs ist, der eine dreidimensionale Armierung (1) umfasst, der einen zu der Armierung (1) inneren Raum definiert, **dadurch gekennzeichnet, dass** die Armierung (1) biologisch resorbierbar ist, dass sie zwei seitliche Öffnungen umfasst, die einen transversalen Durchgang bilden, der das Einsetzen eines Gefäßstiels erlaubt und dass sie ferner mindestens zwei biologisch resorbierbare Textillagen (31; 61) umfasst, die imstande sind, in dem zur Armierung (1) inneren Raum aufeinandergestapelt zu sein.

2. Vorrichtung nach Anspruch 1, **dadurch gekennzeichnet, dass** die Armierung (1) eine Wand aufweist, die einen Rand (13) und eine Vielzahl von Durchbrüchen (15) definiert, die auf der Wand verteilt sind und/oder einen offenen Abschnitt, der in die Wand eingearbeitet ist und sich oberhalb des Randes (13) der Armierung erstreckt, wobei sich der offene Abschnitt (16) ab der Spitze der Kuppel erstreckt, wenn die Armierung (1) etwa die Form einer Kuppel hat.

3. Vorrichtung nach Anspruch 1, **dadurch gekennzeichnet, dass** die Armierung (1) verbundene Bögen (3, 5) aufweist, deren freie Enden sich in derselben Ebene befinden und vorzugsweise mindestens eine erste Gruppe äußerer Bögen (3) und eine zweite Gruppe innerer Bögen (5), die sich in dem von den äußeren Bögen (3) definierten Raum befinden, wobei sich die freien Enden der Bögen der ersten und zweiten Gruppe in derselben Ebene befinden.

4. Vorrichtung nach einem der vorangehenden Ansprüche, **dadurch gekennzeichnet, dass** das Textil aus einer Verflechtung von mindestens zwei Fäden gebildet ist, dass das Textil eine dreidimensionale Struktur bildet, die mindestens zwei übereinanderliegende Oberflächenschichten aufweist, die die zwei gegenüberliegenden Flächen des Textils bilden, dass die Fäden die Oberflächenschichten verbinden, dass die verflochtenen Fäden Poren bilden, die die Dicke des Textils durchqueren und dass das Textil vorzugsweise Verbindungspunkte zwischen den Fäden aufweist.

5. Vorrichtung nach Anspruch 4, **dadurch gekennzeichnet, dass** das Textil ferner mindestens eine Zwischenschicht aufweist, die zwischen den Oberflächenschichten angeordnet ist, dass die Fäden die Oberflächenschichten mit der Zwischenschicht verbinden, dass die verflochtenen Fäden Öffnungen auf der Zwischenschicht bilden, dass vorzugsweise die Öffnungen der Zwischenschicht nicht mit den Öffnungen der gegenüberliegenden Flächen des Textils übereinstimmen und dass das Textil vorzugsweise Verbindungspunkte zwischen den Fäden aufweist.

6. Vorrichtung nach einem der vorangehenden Ansprüche, **dadurch gekennzeichnet, dass** das Textil Poren mit zerstreuten Durchmessern aufweist, wobei das arithmetische Mittel des Durchmessers der Poren etwa gleich oder kleiner als 3,5 und etwa gleich oder größer als 1,5 und vorzugsweise etwa gleich 2 ist und dass 75 % der Poren einen Durchmesser aufweisen, der etwa gleich oder größer als 2 und etwa gleich oder kleiner als 3,5 ist.

7. Vorrichtung nach einem der vorangehenden Ansprüche, **dadurch gekennzeichnet, dass** das Textil eine Bruchverformung in Maschinenrichtung und/oder oder in der Querrichtung von 50 % aufweist.

8. Vorrichtung nach einem der vorangehenden Ansprüche, **dadurch gekennzeichnet, dass** das Textil aus mindestens zwei Fäden mit unterschiedlichem Durchmesser gebildet ist und dass das Verhältnis der Durchmesser der Fäden insbesondere zwischen 2 und 3 liegt.

9. Vorrichtung nach einem der vorangehenden Ansprüche, **dadurch gekennzeichnet, dass** das Textil eine Luftdurchlässigkeit von über oder gleich 10.000 L/m$^2$ und eine Dicke von über oder gleich 0,50 mm aufweist.

10. Vorrichtung nach einem der vorangehenden Ansprüche, **dadurch gekennzeichnet, dass** sie einen Stapel (3) aus mindestens zwei Textillagen (31; 61) aufweist, die zwischen sich eine Schicht von Zellen (33) umfasst, die aus den Adipozyten, den Zellen, die imstande sind, sich in Adipozyten zu differenzieren und den Mischungen aus diesen beiden Zelltypen ausgewählt sind und dass der Stapel in der Armierung untergebracht ist und diese mindestens teilweise und vorzugsweise teilweise ausfüllt.

11. In vitro-Methode zur Herstellung eines Implantats, das imstande ist, im Körper einer Person als Ersatz und/oder zur Vergrößerung eines Weichgewebevolumens angeordnet zu sein, **dadurch gekennzeichnet, dass**:

    - eine Armierung (1) bereitgestellt wird, die eine Größe und eine Form aufweist, die zuvor festgelegt wurden, wobei die Armierung dreidimensional (1) ist, einen inneren Raum definiert, biologisch resorbierbar ist und zwei seitliche Öffnungen umfasst, die einen transversalen Durchgang bilden, der das Einsetzen eines Gefäßstiels erlaubt;
    - mindestens zwei Lagen (31) mindestens eines Textils bereitgestellt werden, das aus einer Verflechtung von mindestens zwei Fäden gebildet ist, die eine dreidimensionale Struktur bilden;
    - mindestens ein erster Stapel (3) aus mindestens zwei der Lagen hergestellt wird, zwischen denen Zellen angeordnet werden, die aus den Adipozyten, den Zellen, die imstande sind, sich in Adipozyten zu differenzieren und den Mischungen aus diesen beiden Zelltypen ausgewählt sind, wobei die Zellen vorzugsweise von der Person stammen;
    - der erste Stapel (3) in der Armierung (1) zwecks Implantation derselben in den Körper einer Person angeordnet wird.

12. Methode nach Anspruch 11, **dadurch gekennzeichnet, dass**:

    - vor oder nach dem Einsetzen des Stapels (3) in die Armierung (1) der Stapel (3) eingeschnitten wird, insbesondere parallel zur Ebene der Lagen (31), etwa im Bereich der seitlichen Öffnungen und insbesondere oberhalb des Randes der Armierung (1).

13. Methode nach Anspruch 11 oder 12, **dadurch gekennzeichnet, dass**

    - ein zweiter Stapel aus mindestens zwei der Lagen hergestellt wird, zwischen denen Zellen angeordnet werden, die aus den Adipozyten, den Zellen, die imstande sind, sich in Adipozyten zu differenzieren und den Mischungen aus diesen beiden Zelltypen ausgewählt sind, wobei die Zellen vorzugsweise von der Person stammen;
    - der erste und zweite Stapel übereinander in der Armierung derart angeordnet werden, dass der transversale Durchgang etwa zwischen dem ersten und dem zweiten Stapel ausmündet.

14. Methode nach einem der vorangehenden Ansprüche, **dadurch gekennzeichnet, dass** ein Textil bereitgestellt wird, das aus einer Verflechtung von mindestens zwei Fäden gebildet ist, dass das Textil eine dreidimensionale Struktur bildet, die mindestens zwei übereinanderliegende Oberflächenschichten aufweist, die die zwei gegenüberliegenden Flächen des Textils bilden, dass die Fäden die Oberflächenschichten verbinden, dass die verflochtenen Fäden Poren bilden, die die Dicke des Textils durchqueren und dass das Textil vorzugsweise Verbindungspunkte zwischen den Fäden aufweist.

15. Methode nach einem der vorangehenden Ansprüche, **dadurch gekennzeichnet, dass** das Textil ferner mindestens eine Zwischenschicht aufweist, die zwischen den Oberflächenschichten angeordnet ist, dass die Fäden die Oberflächenschichten mit der Zwischenschicht verbinden, dass die verflochtenen Fäden Öffnungen auf der Zwischenschicht bilden, dass vorzugsweise die Öffnungen der Zwischenschicht nicht mit den Öffnungen der gegenüberliegenden Flächen des Textils übereinstimmen und dass das Textil vorzugsweise Verbindungspunkte zwischen den Fäden aufweist.

**Claims**

1. A device capable of being implanted into the body of a subject to form an implant capable of replacing and/or increasing a soft tissue volume, said device being of the type comprising a three-dimensional framework (1) which defines a space internal to said framework (1), **characterised in that** said framework (1) is bio-resorbable, **in that** it comprises two side openings forming a transverse passage for inserting a vascular pedicle and **in that** it further comprises at least two bio-resorbable textile sheets (31; 61) capable of being stacked one upon the other into said space internal to said framework (1).

2. The device according to claim 1, **characterised in that** said framework (1) includes a wall which defines an edge (13) and a plurality of perforations (15) distributed on said wall and/or an open portion provided in said wall and extending above said edge (13) of said framework, said open portion (16) extending from the top of the dome when said framework (1) is substantially of a dome shape.

3. The device according to claim 1, **characterised in that** said framework (1) includes assembled arches (3, 5) the free ends or which are located in a same plane and preferably at least a first group of external arches (3) and a second group of internal arches (5), located in the space defined by said external arches (3), the free ends of said arches of said first and second groups being located in a same plane.

4. The device according to any of the preceding claims, **characterised in that** said textile is formed by an interleave of at least two threads, **in that** said textile forms a three-dimensional structure which includes at least two superimposed surface layers, forming the two opposite faces of said textile, **in that** said threads connect said surface layers, **in that** said interleaved threads form pores which pass through the thickness of said textile and **in that** said textile preferably includes connecting stitches between said threads.

5. The device according to claim 4, **characterised in that** said textile further includes at least one intermediate layer between said surface layers, **in that** said threads connect said surface layers to said intermediate layer, **in that** said interleaved threads form openworks on said intermediate layer, **in that**, preferably, said openworks of said intermediate layer do not correspond with said openworks of said opposite faces of said textile and **in that** said textile preferably includes connecting stitches between said threads.

6. The device according to any one of the preceding claims, **characterised in that** said textile includes pores of dispersed diameters, the arithmetic mean of the diameter of said pores is substantially equal or less than 3.5 and substantially equal or greater than 1.5 and preferably substantially equal to 2 and **in that** 75% of said pores have a diameter substantially equal or greater than 2 and substantially equal or less than 3.5.

7. The device according to any one of the preceding claims, **characterised in that** said textile has a strain at failure in the machine direction and/or in the cross direction less than 50%.

8. The device according to any one of the preceding claims, **characterised in that** said textile is formed by at least two threads of different diameter and **in that** the ratio of the diameters of the threads is especially of between 2 and 3.

9. The device according to any one of the preceding claims, **characterised in that** said textile has an air permeability greater than or equal to 10.000 L/m$^2$ and a thickness greater than or equal to 0.50 mm.

10. The device according to any of the preceding claims, **characterised in that** it includes a stack (3) of at least two textile sheets (31; 61) comprising in between a layer of cells (33) selected from adipocytes, cells capable of differentiate into adipocytes and mixtures of these two types of cells and **in that** said stack is housed into said framework and fills it at least partially and preferably partially.

11. An in vitro method for manufacturing an implant capable of being disposed into the body of a subject for replacing and/or increasing a soft tissue volume, **characterised by**:

- providing a framework (1) having a size and a shape determined beforehand; said framework being three-dimensional (1) defining an internal space, being bio-resorbable, and comprising two side openings forming a transverse passage for inserting a vascular pedicle;
- providing at least two sheets (31) of at least one textile formed by an interleave of at least two threads which form a three-dimensional structure;

- forming at least a first stack (3) of at least two of said sheets between which cells selected from adipocytes, cells capable of differentiate into adipocytes and mixtures of the two types of cells are deposited, said cells preferably coming from said subject;
- disposing said first stack (3) into said framework (1) for implanting the latter into the body of a subject.

12. A method according to claim 11, **characterised by**:

- before or after inserting said stack (3) into said framework (1), making an incision in said stack (3), especially in parallel to the plane of said sheets (31), substantially at said side openings and especially above said edge of said framework (1).

13. The method according to claim 11 or 12, **characterised by**

- performing a second stack of at least two of said sheets between which cells selected from adipocytes, cells capable of differentiate into adipocytes and mixtures of these two types of cells are deposited, said cells preferably coming from said subject;
- disposing said first and second stacks one upon the other in said framework, so that said transverse passage substantially opens out between said first and said second stack.

14. A method according to any of the preceding claims, **characterised by** providing a textile formed by an interleave of at least two threads, in that said textile forms a three-dimensional structure which includes at least two superimposed surface layers, forming the two opposite faces of said textile, in that said threads connect said surface layers, in that said interleaved threads form pores which pass through the thickness of said textile and in that said textile preferably includes, connecting stitches between said threads.

15. The method according to any of the preceding claims, **characterised in that** said textile further includes at least one intermediate layer disposed between said surface layers, **in that** said threads connect said surface layers to said intermediate layer, **in that** said interleaved threads form openworks on said intermediate layer, **in that** preferably said openworks of said intermediate layer do not correspond with said openworks of said opposite faces of said textile and **in that** said textile preferably includes connecting stitches between said threads.

**FIG. 1a**

**FIG. 1b**

**FIG. 2**

**FIG. 3A**

FIG. 3B

FIG. 3C

FIG. 4

FIG. 5

A                                    B

FIG. 6

A                    B

FIG. 7

A

B

FIG. 8

**FIG. 9**

**FIG. 10**

FIG. 11

## RÉFÉRENCES CITÉES DANS LA DESCRIPTION

**Documents brevets cités dans la description**

- WO 0166039 A1
- US 5545217 A1
- US 20140135925 A1
- US 2014135925 A1

**Littérature non-brevet citée dans la description**

- **M. W. FINDLAY.** Tissue-engineered Breast Reconstruction : Bridging the Gap toward Large-Volume Tissue Engineering in Humans. *PRSJournal,* 2011, vol. 128 (6), 1206-1215
- **W.A. MORRISON.** Creation of a Large Adipose Tissue Construct in Humans Using a Tissue-engineering Chamber: A Step Forward in the Clinical Application of Soft Tissue Engineering. *EbioMedecine,* Avril 2016